(19) Europäisches Patentamt
European Patent Office
Office européen des brevets

(11) EP 0 694 299 B1

(12) EUROPEAN PATENT SPECIFICATION

(45) Date of publication and mention of the grant of the patent:
**19.09.2001 Bulletin 2001/38**

(51) Int Cl.$^7$: **A61K 31/135**

(21) Application number: **95110311.8**

(22) Date of filing: **03.07.1995**

(54) **The use of( a) bicycloheptane derivative(s)**

Verwendung vom einem Bicycloheptan-Derivat

Utilisation d'un dérivé de bicycloheptane

(84) Designated Contracting States:
**AT CH DE DK ES IE LI NL SE**

(30) Priority: **01.07.1994 HU 9401968**

(43) Date of publication of application:
**31.01.1996 Bulletin 1996/05**

(73) Proprietor: **EGIS GYOGYSZERGYAR RT.**
**1106 Budapest (HU)**

(72) Inventors:
- **Budai, Zoltán, Dr.**
  **H-1023 Budapest (HU)**
- **Cacsályi, István**
  **H-1021 Budapest (HU)**
- **Gábor Szénási, dipl. bio.**
  **H-1035 Budapest (HU)**
- **Mezei, Tibor, Dr.**
  **H-1221 Budapest (HU)**
- **Kovács, Aniko, Dr.**
  **H-1132 Budapest (HU)**
- **Blasko, Gábor, Dr.**
  **H-1113 Budapest (HU)**
- **Szemerédi, Katalin, Dr.**
  **H-2011 Budakalász (HU)**
- **Simig, Gyula, Dr.**
  **H-1126, Budapest (HU)**
- **Petocz, Lujza, Dr.**
  **H-1084 Budapest (HU)**
- **Reiter née Esses, Klára, Dr.**
  **H-1022 Budapest (HU)**

(74) Representative: **Beszédes, Stephan G., Dr.**
**Patentanwalt**
**Postfach 11 68**
**85201 Dachau (DE)**

(56) References cited:
**EP-A- 0 446 133** **WO-A-94/18229**
**US-A- 4 342 762**

- **ARZNEIM.-FORSCH., vol. 39, no. 3, 1989 pages 295-297, I. KOVACS ET AL. 'INHIBITION OF HIGH-AFFINITY SYNAPTOSOMAL UPTAKE OF gamma-AMINOBUTYRIC ACID BY A BICYCLO-HEPTANE DERIVATIVE'**
- **ANNALES PHARMACEUTIQUES FRANCAISE, vol. 24, no. 3, 1966 pages 185-190, R. BARONNET ET AL. 'ETUDE D'ETHERS OXYDES AMINES D'ALCOOLS TERPENIQUES'**
- **THE LANCET, vol. 334, no. 8, 1989 page 167 C. BEGLINDER ET AL. 'TREATMENT OF BILIARY COLIC WITH LOXIGLUMIDE'**
- **R.S.L. CHANG ET AL.: "Tifluadom, a kappa-opiate agonist, acts as a peripheral cholecystokinin receptor antagonist", NEUROSCIENCE LETTERS, , , vol. 72, no. 2, pages 211 to 214**
- **T.C. KAMILARIS ET AL.: "Cholecystokinin-Octapeptide stimulates Hypothalamic-Pituitary-Adrenal Function in rats: Role of Corticotropin-Releasing Hormone", ENDOCRINOLOGY, , , vol. 130, no. 4, pages 1764 to 1774**
- **J.N. CRAWLEY: "Clarification of the Behavioral Functions of Peripheral and Central Cholecystokinin: Two Separate peptide Pools", PEPTIDES, , , vol. 6, no. 2, pages 129 to 136**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European Patent Convention).

EP 0 694 299 B1

## Description

The invention concerns a novel medical use of [a] bicycloheptane derivative(s).

It is known that bicycloheptane derivatives of general formula

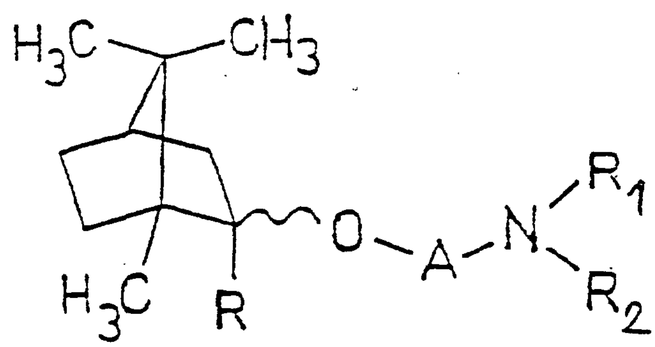

O,

wherein

| | |
|---|---|
| $R_1$ and $R_2$, | which may be the same or different, represent $C_{1-5}$ alkyl groups or $C_{3-6}$ cycloalkyl groups or they form together with the nitrogen atom to which they are attached a heterocyclic ring containing 4 to 7 carbon atoms and optionally a further hetero atom, e.g. an oxygen, sulfur or nitrogen atom, this latter optionally being substituted by a $C_{1-3}$ alkyl, benzyl or phenyl group, |
| R | means a phenyl, phenyl-($C_{1-3}$ alkyl) or thienyl group, optionally substituted by one or more halogen or $C_{1-3}$ alkoxy substituent(s), |
| A | represents a $C_{2-5}$ straight or branched alkylene chain and |
| ~ | represents a valence bond of β configuration, |

have anticonvulsive, motility inhibiting and analgesic activities and furthermore potentiate the narcosis induced by 5-(1'-cyclohexenyl)-1,5-di-(methyl) -barbituric acid [hexobarbital]. In case of certain compounds, the above main activities are supplemented by weak antiserotonin, gastrointestinal peristaltic inhibiting and antiinflammatory effects (US-A 4,342,762).

In Arzneim.-Forsch./Drug res. 39 (I), No. 3 (1989), pages 295 to 297 anxiolytic and anticonvulsive activities of dia(-)2-phenyl-2-(dimethylaminoethoxy)-1R-1,7,7-trimethyl--bicyclo[2.2.1]heptane hemifumarate have been described.

From Annales pharmaceutiques francaises, Vol. 24, No. 3, 1966, pages 185 to 190 localanaesthetic and neurotropic and myotrapic antispasmodic activities of polycyclic compounds of the terpene type substituted by a (dialkylamino-ethoxy or heterocyclic-N-ethoxy group, such as d-bornyloxy-2--diethylamino-1-ethane have been known.

Furthermore it has been known that the peripheral cholecystokinin (CCK) system play an important role in pain (see EP-A-446 133 and Neuroscience Letters, Vol. 72(2), 1986, pages 211 to 214 [R.S.L. Chang et al.]), movement disorders, including Parkinson's disease (see WO-A-94 18 229). Moreover, peripherally administered CCK is anxiogenic and promotes the occurrence of acute panic attacks (see Endocrinology, Vol. 130(4), 1992, pages 1764 to 1774 [T.C. Kamilaris et al.]). Peripherally administered CCK does not pass the blood-brain barrier and does not get into the brain (see Endocrinology, Vol. 130(4), 1992, pages 1764 to 1774 [T.C. Kamilaris et al.] and Peptides, Vol. 6, 1985, pages 129 to 136 [J.N. Crawley]). It has been asserted that all behavioural actions of peripherally administered CCK are initiated at peripheral CCK receptors. However, from the disclosure of an effect mediated via $CCK_A$ receptors it does not follow necessarily that it is of peripheral origin. Rather it would have to be correctly proven experimentally.

The spastic contraction of the cholecyst occurs, in most instances, if in the gallbladder there are gallstones blocking the bile duct. In case of obstruction, the biliary flow is reduced or stopped, the consequence of which is an elevated pressure in the gallbladder leading to a very strong pain.

Cholelithiasis is a disease that occurs frequently, about 10% of the population is affected by it (W. C. Bowman and M. J. Rand, Textbook of Phalmacology, Blackwell Scientific Publications, Oxford, 1980, p. 2 610). Three main types

of gallstones are distinguished in the literature: gallstones of cholesterol type, pigment type and mixed type. The gallstones of mixed type occur most often. In case of women, the occurence of gallstones is twice so high than in case of men. Also in persons having overweight, the occurence of gallstones is more frequent.

In cholelithiasis, the chemotherapy of spastic states is not settled at present since the available drugs do not inhibit the spastic contraction of gallbladder in a selective way.

In general, the surgical intervention (i.e. the removal of the gallstone or cholecyst) is preferred. Chemotherapy includes the administration of nitroglycerol, atropin, analgesics and spasmolytics (J. Knoll, Gyógyszertan, Medicina, Budapest, 1983, p. 372; I. Magyar, Rövid belgyógyászat, Medicina, Budapest, 1985, p. 554; Goodman and Gilman's, The pharmacological basis of therapeutics, Macmillan, New York, 1985, p. 822).

Because of their extremely wide spectrum of effect, atropin and nitroglycerol are rarely employed in practice since a great number of other effects (i.e. side effects) occur, too.

Hence the problem underlying to the invention is to create a novel use of known compounds for preparing superior medicaments for the treatment of diseases and disorders which are connected with the influence on the peripheral cholecystokinin (CCK) system, namely the spastic contraction of gallbladder, such as bilious attack and biliary colic, which have reduced harmful side effects.

Surprisingly the above problem has been solved by the recognition on which the invention is based as well as by bringing it into practice.

The invention is based on the recognition that bicycloheptane derivatives exert their effect in the prevention or treatment of the spastic contraction of gallbladder, such as bilious attack and biliary colic, which have reduced harmful side effects through the inhibition of the peripheral cholecystokinin (CCK) system.

Cholecystokinin is released from the secretory cells being at the distal part of duodenum in the mucosa. In the first place, the release of cholecystokinin is caused by the presence of lipids and essential amino acids. Due to the blood circulation, cholecystokinin reaches the cholecyst giving rise to the contraction thereof, thus, the discharge of bile is realized. Relying upon the results of investigations performed on human beings, it seems that, under physiological conditions, CCK is responsible for the discharge of the gallbladder in 80%. Thus, if the effect of CCK that releases as a consequence of a physiological stimulus can be blocked, then the contraction of the gallbladder can be prevented - and this is the aim in the present case. In fact, bilious attack can be considered as an extreme contraction (W. C. Bowman and M. J. Rand, Textbook of Pharmacology, Blackwell Scientific Publications, Oxford, 1980, pp. 2 520 to 2 521) that is prevented by the receptor antagonists $CCK_A$ (Beglinget et al., Lancet, Vol. 334, No. 8 655, 1989, p. 167).

Surprisingly, it has been found that the compounds defined as follows prevent the spastic contraction of the gallbladder and have activity against cholelithiasis and acute pancreatitis.

Hence the subject matter of the invention is the use of

[a] bicycloheptane derivative(s) of formula

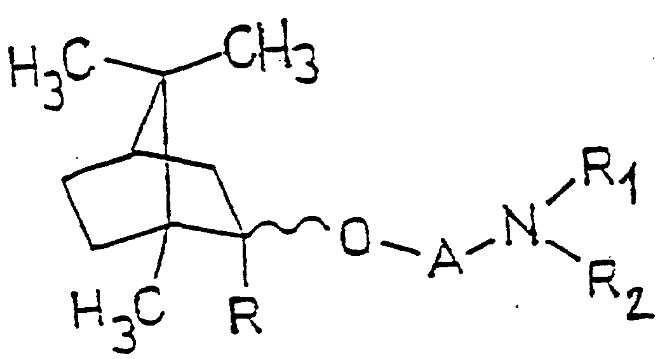

I ,

wherein

| | |
|---|---|
| R | represents a phenyl or a benzyl group, |
| $R_1$ and $R_2$ , | which may be the same or different stand for straight or branched chained alkyl groups having from 1 to 4 carbon atom(s) or one of $R_1$ and $R_2$ is hydrogen and the other is a straight or branched chained alkyl group having from 1 to 4 carbon atom(s), |
| A | denotes a straight or branched chained alkylene group having from 2 to 4 carbon atoms and |

~ represents a valence bond,

and [an] N-oxide(s) as well as [an] optical isomer(s) thereof and mixtures of the optical isomers and [an] acid addition salt(s) and quaternary ammonium derivative(s) of the bicycloheptane derivatives of formula I as well as [an] optical isomer(s) thereof and mixtures of the optical isomers for preparing medicaments for the prevention or treatment of diseases and disorders which are connected with the influence on the peripheral cholecystokinin (CCK) system, selected from spastic contraction of the gallbladder and cholelithiasis as well as acute pancreatitis.

The use according to the invention has the therapeutical advantage that it is able to influence, in a selective manner, the system being in direct connection with the gallbladder, thus, in addition to enhancing the efficaciousness of the therapy, the probability of the appearance of harmful side effects is reduced.

Examples for alkyl groups having from 1 to 4 carbon atoms are methyl, ethyl, n-propyl and isopropyl groups.

It is preferred that as bicycloheptane derivative(s) such in which the alkyl group(s) which is/are represented by $R_1$ and/or $R_2$ has/have 1 or 2, particularly 1, carbon atom(s) is/are employed. Above all in case that $R_1$ and $R_2$ both stand for alkyl groups they represent methyl groups.

Furthermore it is preferred that as bicycloheptane derivative(s) such in which the alkylene group represented by A has 2 or 3, particularly 2, carbon atoms is/are employed. Most preferably A stands for an ethylene, propylene or 2--(methyl)-propylene group.

Formula I of the bicycloheptane derivatives used according to the invention includes all possible optical isomers und any mixtures thereof. The bicycloheptane derivatives of general formula I can correspond to configurations (1R,2S,4R), (1S,2R,4S) or (1RS,2RS,4RS); a preferred configuration is (1R,2S,4R).

Suitably the acid addition salts of the bicycloheptane derivatives of general formula I are pharmaceutically acceptable ones. They can have been formed with inorganic acids, e.g. hydrohalogenic acids, such as hydrochloric acid or hydrobromic acid, sulfuric acid, phosphoric acid or nitric acid, or organic acids, e.g. tartaric acid, succinic acid, malic acid, maleic acid, fumaric acid, citric acid, lactic acid, methanesulfonic acid or p-toluenesulfonic acid, those with fumaric acid being preferred.

The quaternary ammonium derivatives of the bicycloheptane derivatives of general formula I can have been formed by reacting the bicycloheptane derivatives of general formula I with alkyl halides, e.g. methyl, ethyl, n-propyl or isopropyl chloride, bromide or iodide.

It is especially preferred that as bicycloheptane derivative(s) (1R,2S,4R)-(-) -2- [phenyl] -2- [2'- (dimethylamino)--ethoxy]-1,7,7-tri-[methyl]-bicyclo[2.2.1]heptane and/or [an] acid addition salt(s), particularly (1R,2S,4R)-(-)-2-[phenyl]--2-[2'- (dimethylamino)-ethoxy] -1,7,7-tri-[methyl]--bicyclo[2.2.1]heptane (E)-2-butenedioate (salt of the former with fumaric acid), is/are employed since these compounds have particularly useful pharmacological properties.

It is also preferred that as bicycloheptane derivative (s) (1R,2S,4R)-(-)-2-[benzyl]-2-[3'-(dimethylamino)-propoxy]--1,7,7-tri-[methyl]-bicyclo[2.2.1]heptane, (1R,2S,4R)-(-)-2-- [benzyl]-2- [2'-(methyl) -3'-(dimethylamino)-propoxy]-1,7,7--tri-[methyl]-bicyclo[2.2.1]heptane, (1RS,2RS,4RS)-2-[phenyl]--2-[2'-(dimethylamino) -ethoxy]-1,7,7-tri-[methyl]--bicyclo[2.2.1]heptane and/or (1S,2R,4S)-(+)-2-[phenyl] -2- [2'-- (dimethylamino) -ethoxy] -1,7,7-tri-[methyl]--bicyclo[2.2.1]heptane and/or [an] acid addition salt(s), particularly the (E) -2-butenedioate(s), thereof is/are employed.

Conveniently the use according to the invention is for preparing as medicaments pharmaceutical compositions, particularly for oral, rectal or parenteral administration. Preferably the use according to the invention is for preparing as medicaments pharmaceutical compositions in the form of tablets, enteric-coated tablets, capsules, dragées, solutions, suspensions, suppositories or injectable solutions.

It is also preferred that the use according to the invention is for preparing as medicaments pharmaceutical compositions which contain from 10 to 100 mg of bicycloheptane derivative(s) in an unit dosage. This is particularly true in case of tablets, enteric-coated tablets, dragées and capsules.

These pharmaceutical compositions can be prepared by conventional methods used in the manufacture of pharmaceutical compositions, suitably by admixing the bicycloheptane derivative (s) of general formula I in a manner known in itself to 1 or more conventional solid and/or liquid pharmaceutical carrier(s) and/or auxiliary material (s) and transforming the mixture obtained into a pharmaceutical composition.

Preferably the above solid pharmaceutical compositions prepared by the use according to the invention comprise silica and/or 1 or more binding agent(s), such as poly-- (vinylpyrrolidone) and/or gelatin. Furthermore 1 or more lubricant(s), such as magnesium stearate, talc and/or sodium laurylsulfate, can be present additionally to the bicycloheptane derivative(s) in the tablets.

In case of aqueous suspensions and/or elixirs suitable for oral treatment prepared by the use according to the invention, the bicycloheptane derivative(s) can be present in mixture with 1 or more different flavouring agent (s), dyestuff(s), emulgator(s) and/or diluent(s), such as water, ethanol, propylene glycol and/or glycerol.

The tablets prepared by the use according to the invention can be manufactured using dry or wet granulation processes. Dragées can be prepared by coating the core in a usual manner. For the preparation of capsules, the suitable mixture is filled into hard or soft gelatin capsules.

The pharmaceutical compositions prepared by the use according to the invention inhibiting the spastic contraction of the gallbladder are, in general, administered in a dose of 0.25 to 40 mg, preferably 1 to 20 mg, for each kg body weight, in 1 to 3 portions, daily. The actual dosage is determined depending on the activity of the bicycloheptane derivative(s), the method of treatment, the state of the patient and other factors, as described by the attending physician.

The daily dose of the bicycloheptane derivatives used according to the invention depends on the conditions of the given case, e.g. the body weight and age of the patient and the severity of the disorder to be treated, and is determined by the physician. The daily dose for an adult patient is, in general, about 1 mg to about 100 mg of bicycloheptane derivative(s).

The effect of the bicycloheptane derivatives used according to the invention is verified on the following test. Known spasmolytics 1-[3',4'-di-(ethoxy)-benzyliden]-6,7-di-- [ethoxy] -1,2,3,4-terra-[hydro] -isoquinoline {drotaverine} and 1- [3',4'-di-(methoxy)-benzyl]-6,7-di-[methoxy] -isoquinoline {papaverine} are employed for comparison. (These spasmolytics are most often used in the therapeutical practice).

The tests were performed on male mice from the strain NMRI in groups consisting of 8 to 12 animals. At the beginning of the test, the animals were weighing 20 to 30 g.

The animals were starved for 24 hours before beginning the first treatment, however, they were allowed to consume water ad libitum. The compounds to be tested and the carrier (0.4% solution of methyl cellulose) were administered perorally in a volume of 10 ml/kg.

45 minutes after this treatment, an emulsion of 30% of yolk in 0.4% methyl cellulose solution was administered perorally. Each mouse consumed 0.5 ml of the emulsion. The animals of the control group consumed 0.5 ml of the carrier i.e. 0.4% methyl cellulose solution.

After 15 minutes, cervicalis dislocatio was employed, the gallbladders were removed and weighed, one by one. The effect of the compounds tested was given as the inhibition of the cholecyst mass decrease induced by yolk, in percentage. From the effects expressed in percentage, $ID_{50}$ values (i.e. doses causing 50% inhibition) were calculated on the basis of dose versus effect correlations by linear regression (Makovec et. al., Pharm. Res. Com., Vol, 19, No. 1, [1987], p. 41). The results obtained are summarized in the following table.

Table

| Compound tested | $ID_{50}$ p.o. in mg/kg |
|---|---|
| (1R,2S,4R)-(-)-2-[Phenyl]-2-[2'-(dimethylamino)-ethoxy]-1,7,7-tri-[methyl] -bicyclo[2.2.1] heptane (E)-2-butenedioate | 7.0 |
| (1S,2R,4S)-(+)-2- [Phenyl] -2- [2'- (dimethylamino)-ethoxy]-1,7,7-tri- [methyl] -bicyclo[2.2.1] heptane (E) -2-butenedioate | higher than 100 |
| (1RS,2RS,4RS)-2-[Phenyl]-2-[2- (dimethylamino)-ethoxy]-1,7,7-tri- [methyl] -bicyclo [2.2.1] heptane (E) -2-butenedioate | 10 to 100 |
| (1R,2S,4R)-(-)-2-[Phenyl]-2-[2'- (dimethylamino)-ethoxy]-1,7,7-tri- [methyl]-bicyclo[2.2.1] heptane-N-oxide (E)-2-butenedioate | higher than 100 |
| (1R,2S,4R)-(-)-2-[Phenyl]-2-[2'- (methylamino)-ethoxy]-1,7,7-tri- [methyl]-bicycle[2.2.1] heptane (E)-2-butenedioate | 10 to 100 |
| (1R,2S,4R)-(-)-2-[Benzyl]-2-[2'- (methyl) -3-di- (methylamino)-propoxy]-1,7,7-tri-[methyl]-bicyclo[2.2.1]heptane (E)-2-butenedioate | 10 to 100 |
| (1R,2S,4R)-(-)-2-[Benzyl]-3-[3'-(dimethylamino)-propoxy]-1,7,7-tri- [methyl]-bicyclo[2.2.1] heptane (E)-2-butenedioate | 10 to 100 |
| 1-[3',4'-di-(Ethoxy)-benzyliden]-6,7-di-[ethaxy]-1,2,3,4-tetra-[hydro]-isoquinoline {Drotaverine} <Reference substance I> | higher than 100 |
| 1-[3',4'-di-(Methoxy)-benzyl]-6,7-di- [methoxy]-isoquinoline {Papaverine} <Reference substance II> | higher than 100 |

From the above comparison it can be seen that, as to activity, the bicycloheptane derivatives used according to the invention for the most part surpass the known spasmolytics used for comparison and at least reach them. Thus, (1R,2S,4R)--(-)-2-[phenyl]-2-[2'-(dimethylamino)-ethoxy]-1,7,7-tri-- [methyl] -bicyclo[2.2.1]heptane (E) -2-butenedioate inhibits the cholecyst contraction induced by yolk at a dose of as low as 7 mg/kg more efficiently by one order of magnitude than the known spasmolytics used for comparison.

Consequently, the pharmaceutical compositions prepared from the bicycloheptane derivatives by the use according to the invention can be effectively employed in clinical patterns which include the CCK system as a pathogen factor, namely the spastic contraction of gallbladder in the first place, furthermore other diseases and disorders, for instance acute pancreatitis.

It is deemed that the spasmolytic mechanism of the bicycloheptane derivatives of general formula I and its derivatives in the treatment of bilious attacks is different from that of the known spasmolytics. Drugs relieving the spastic state of the smooth muscle are called spasmolytics. The smooth muscle spasmolytics, such as 1- [3',4'-di-(methoxy)--benzyl]-6,7-di-[methoxy]-isoquinoline <papaverine> or 1-- [3',4'-di-(ethoxy)-benzyliden]-6,7-di-[ethoxy]-1,2,3,4-tetra--[hydro]-isoquinoline <drotaverine>, are not able to relieve the spasms of the skeletal muscle.

The bicycloheptane derivatives used according to the invention do not show a direct spasmolytic effect in tests with isolated organs, however, they can relieve the bilious attacks induced experimentally. This contradiction is explained by the fact that the bicycloheptane derivatives used according to the invention influence the CCK system. This effect of these substances is novel and surprising for the expert.

The invention is further elucidated by the following Examples.

Example 1

Tablet containing 25 mg of active ingredient

One tablet contains:

| | |
|---|---|
| (1R,2S,4R)-(-)-2-[phenyl]-2-[2'-(dimethylamino)-ethoxy]-1,7,7-tri-[methyl]-bicyclo[2.2.1]heptane (E)-2-butenedioate | 25.0 mg |
| maize starch | 97.0 mg |
| poly-(vinylpyrrolidone) | 75.0 mg |
| magnesium stearate | 3.0 mg |
| | 200.0 mg |

Preparation:

A mixture of the active ingredient and maize starch is granulated by wetting with a 15% by weight aqueous poly-(vinylpyrrolidone) solution and drying at 40 to 45°C. The granules are dried again, then mixed with magnesium stearate and tabletted. The weight of a tablet is 200.0 mg.

Example 2

Dragée containing 25 mg of active ingredient

One dragée core contains:

| | |
|---|---|
| (1R,2S,4R)-(-)-2-[phenyl]-2-[2'-(dimethylamino)-ethoxy]-1,7,7-tri-[methyl]-bicyclo[2.2.1]heptane (E)-2-butenedioate | 25.0 mg |
| maize starch | 245.0 mg |
| gelatin | 8.0 mg |
| talc | 18.0 mg |
| magnesium stearate | 4.0 mg |
| | 300.0 mg |

Preparation:

A mixture of the active ingredient and maize starch is wetted with a 10% aqueous gelatin solution, then granulated by passing it through a sieve, and dried at 40 to 45°C. The dry granules are again passed through a sieve, homogenized with talc and magnesium stearate, and compressed to dragee cores weighing 300.0 mg each.

The dragée cores obtained are coated with a layer consisting of sugar and talc in a manner known per se. The dragees obtained are polished with beeswax.

Example 3

Dragée containing 50 mg of active ingredient
One dragée core contains:

| | |
|---|---|
| (1RS,2RS,4RS)-2-[phenyl]-2-[2'-(dimethylamino)-ethoxy]-1,7,7-tri-[methyl]-bicyclo[2.2.1]heptane (E)-2-butenedioate | 50.0 mg |
| lactose | 94.0 mg |
| poly-(vinylpyrrolidone) | 4.0 mg |
| magnesium stearate | 2.0 mg |
| | 150.0 mg |

Preparation:

The granules and dragée cores are prepared as described in Example 2. The weight of a dragée core is 150.0 mg. Then, the cores are coated as given in Example 2 to obtain dragées.

Example 4

Gelatin capsule containing 25 mg of active ingredient
One capsule contains:

| | |
|---|---|
| (1R,2S,4R)-(-) -2- [phenyl] -2- [2' - (methylamino)-ethoxy]-1,7,7-tri-[methyl]-bicyclo[2.2.1]heptane (E) -2-butenedioate | 25.0 mg |
| maize starch | 265.0 mg |
| silicium dioxide [Aerosil®] | 6.0 mg |
| magnesium stearate | 4.0 mg |
| | 300.0 mg |

Preparation:

The ingredients are homogenized, then filled into gelatin capsules of suitable size.

Example 5

Injectable solution containing 25 mg of (1R,2S,4R)-(-)-2-- [phenyl] -2-[2'-(dimethylamino) -ethoxy] -1,7,7-tri-[methyl]--bicyclo[2.2.1]heptane (E) -2-butenedioate
One ampoule contains:
active ingredient of formula I 25.0 mg
in 1 ml of water which was distilled twice.

## Claims

**1.** The use of [a] bicycloheptane derivative(s) of formula

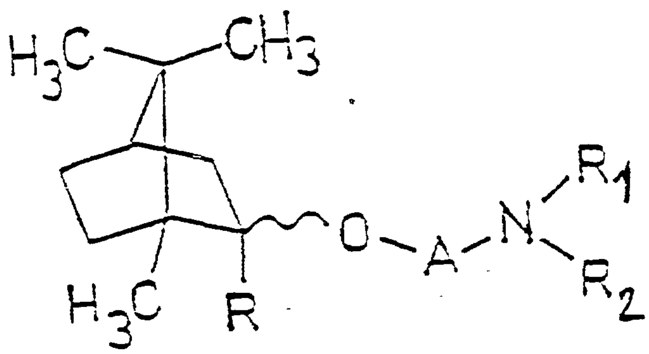

I ,

wherein

R represents a phenyl or a benzyl group,

$R_1$ and $R_2$, which may be the same or different stand for straight or branched chained alkyl groups having from 1 to 4 carbon atcm(s) or one of $R_1$ and $R_2$ is hydrogen and the other is a straight or branched chained alkyl group having from 1 to 4 carbon atcm(s),

A denotes a straight or branched chained alkylene group having from 2 to 4 carbon atoms and

~ represents a valence bond,

and [an] N-oxide(s) as well as [an] optical isomer(s) thereof and mixtures of the optical isomers and [an] acid addition salt(s) and quaternary ammonium derivative(s) of the bicycloheptane derivatives of formula I as well as [an] optical isomer(s) thereof and mixtures of the optical isomers for preparing medicaments for the prevention or treatment of diseases and disorders which are connected with the influence on the peripheral cholecystokinin (CCK) system, selected from spastic contraction of the gallbladder and cholelithiasis as well as acute pancreatitis

**2.** The use according to claim 1, **characterized in that** as bicycloheptane derivative(s) such in which the alkyl group (s) which is/are represented by $R_1$ and/or $R_2$ has/have 1 or 2 carbon atom(s) is/are employed.

**3.** The use according to claim 1 or 2, **characterized in that** as bicycloheptane derivative(s) such in which the alkylene group represented by A has 2 or 3 carbon atoms is/are employed.

**4.** The use according to claims 1 to 3, **characterized in that** as bicycloheptane derivative(s) (1R,2S,4R)-(-)-2-[phenyl]-2-[2'-(dimethylamino)-ethoxy]-1,7,7-tri-[methyl]--bicyclo[2.2.1]heptane and/or [an] acid addition salt(s), particularly (1R,2S,4R)-(-)-2-[phenyl]-2-[2'-- (dimethylamino)-ethoxy]-1,7,7-tri-[methyl]--bicyclo[2.2.1]heptane (E)-2-butenedioate, is/are employed.

**5.** The use according to claims 1 to 4, **characterized in that** as bicycloheptane derivative(s) (1R,2S,4R)-(-) -2- [benzyl] -2- [3'-(dimethylamino) -propoxy]--1,7,7-tri-[methyl]-bicyclo[2.2.1]heptane, (1R,2S, 4R)-- (-) -2- [benzyl]-2- [2'-(methyl) -3'-(dimethylamino)--propoxy]-1,7,7-tri-[methyl]-bicyclo[2.2.1]heptane, (1RS, 2RS,4RS)-2- [phenyl]-2- [2'-(dimethylamino)-ethoxy]--1,7,7-tri-[methyl]-bicyclo[2.2.1]heptane and/or (1S,2R,4S)-(+)-2- [phenyl] -2- [2'-(dimethylamino) -ethoxy]--1,7,7-tri-[methyl]-bicyclo[2.2.1]heptane and/or [an] acid addition salt(s), particularly the (E)-2--butenedioate(s), thereof is/are employed.

**6.** The use according to claims 1 to 5, **characterized in that** it is for preparing as medicaments pharmaceutical compositions, particularly for oral, rectal or parenteral administration.

**7.** The use according to claims 1 to 6, **characterized in that** it is for preparing as medicaments pharmaceutical compositions in the form of tablets, enteric-coated tablets, capsules, dragees, solutions, suspensions, suppositories or injectable solutions.

**8.** The use according to claims 1 to 7, **characterized in that** it is for preparing as medicaments pharmaceutical compositions which contain from 10 to 100 mg of bicycloheptane derivative(s).

## Patentansprüche

**1.** Verwendung von [einem] Bicycloheptanderivat(en) der Formel:

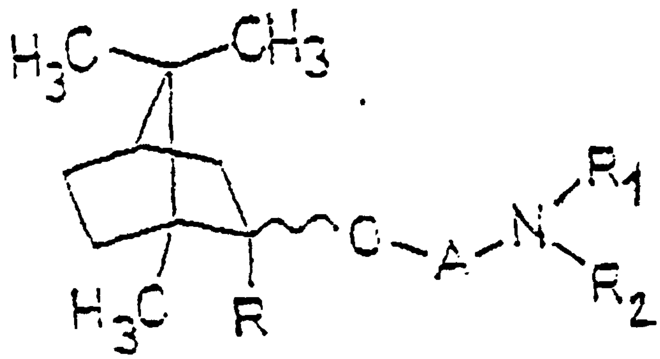

I,

worin

| | |
|---|---|
| R | eine Phenyl- oder eine Benzylgruppe bedeutet, |
| $R_1$ und $R_2$, | die gleich oder verschieden sein können, für geradkettige oder verzweigtkettige Alkylgruppen mit 1 bis 4 Kohlenstoffatomen stehen oder eines von $R_1$ und $R_2$ Wasserstoff ist und das andere eine geradkettige oder verzweigtkettige Alkylgruppe mit 1 bis 4 Kohlenstoffatomen ist, |
| A | eine geradkettige oder verzweigtkettige Alkylengruppe mit 2 bis 4 Kohlenstoffatomen bedeutet und |
| - | für eine Valenzbindung steht, |

und von [einem] N-oxid(en) sowie von [einem] optischen Isomer(en) davon und Mischungen der optischen Isomeren und von [einem] Säureadditionssalz(en) und von quaternärem(n) Ammoniumderivat(en) der Bicycloheptanderivate der Formel I sowie von [einem] optischen Isomer(en) davon und Mischungen der optischen Isomeren zur Herstellung von Medikamenten zur Vorbeugung oder Behandlung von Krankheiten und Beschwerden, die mit dem Einfluss auf das periphere Cholecystokinin (CCK-)-System verbunden sind, gewählt aus einer krampfartigen Kontraktion der Gallenblase und Cholelithiasis sowie akuter Entzündung der Bauchspeicheldrüse bezeichnet.

**2.** Verwendung gemäss Anspruch 1, **dadurch gekennzeichnet, dass** als Bicycloheptanderivat(e) solche, bei welchen die Alkylgruppe(n), die durch $R_1$ und/oder $R_2$ angegeben ist/sind, 1 oder 2 Kohlenstoffatom(e) aufweisen, verwendet wird/werden.

**3.** Verwendung gemäss Anspruch 1 oder 2, **dadurch gekennzeichnet, dass** als Bicycloheptanderivat(e) solche, bei welchen die Alkylengruppe(n), angegeben durch A, 2 oder 3 Kohlenstoffatome aufweist, verwendet wird/werden.

**4.** Verwendung gemäss Anspruch 1 bis 3, **dadurch gekennzeichnet, dass** als Bicycloheptanderivat(e) (1R, 2S, 4R)-(-)-2-[Phenyl]-2-[2'-(dimethylamino)-ethoxy]-1,7,7-tri-[methyl]-bicyclo[2.2.1]-heptan und/oder [ein] Säureadditionssalz(e), insbesondere (1R, 2S, 4R)-(-)-2-[Phenyl]-2-[2'-(dimethylamino)-ethoxy]-1,7,7-tri-[methyl]-bicyclo [2.2.1]-heptan-(E)-2-butendioat verwendet wird/werden.

**5.** Verwendung gemäss Anspruch 1 bis 4, **dadurch gekennzeichnet, dass** als Bicycloheptanderivat(e) (1R, 2S, 4R)-(-)-2-[Benzyl]-2-[3'-(dimethylamino)-propoxy]-1,7,7-tri-[methyl]-bicyclo-[2.2.1]-heptan, (1R, 2S, 4R)-(-)-2-[Benzyl]-2-[2'-(methyl)-3'-(dimethylamino)-propoxy]-1,7,7-tri-[methyl]-bicyclo-[2.2.1]-heptan, (1RS, 2RS, 4RS)-2-[Phenyl]-2-[2'-dimethylamino)-ethoxy]-1,7,7-tri-[methyl]-bicyclo[2.2.1]-heptan und/oder (1S, 2R, 4S)-(+)-2-[Phenyl]-2-[2'-(dimethylamino)-ethoxy]-1,7,7-tri-[methyl]-bicyclo-[2.2.1]-heptan und/-oder [ein] (E)-2-Butendioat(e) davon verwendet wird/werden.

6. Verwendung gemäss Anspruch 1 bis 5, **dadurch gekennzeichnet, dass** sie der Herstellung als pharmazeutische Medikamentenzusammensetzungen, insbesondere für die orale, rektale oder parenterale Verabreichung, dient.

7. Verwendung gemäss Anspruch 1 bis 6, **dadurch gekennzeichnet, dass** sie der Herstellung als pharmazeutische Medikamentenzusammensetzungen in der Form von Tabletten, enterisch beschichteten Tabletten, Kapseln, Dragées, Lösungen, Suspensionen, Zäpfchen oder injizierbaren Lösungen dient.

8. Verwendung gemäss Anspruch 1 bis 7, **dadurch gekennzeichnet, dass** sie der Herstellung als pharmazeutische Medikamentenzusammensetzungen, die 10 bis 100 mg Bicycloheptanderivat(e) enthalten, dient.

**Revendications**

1. Utilisation d'un ou de dérivés de bicycloheptane de formule :

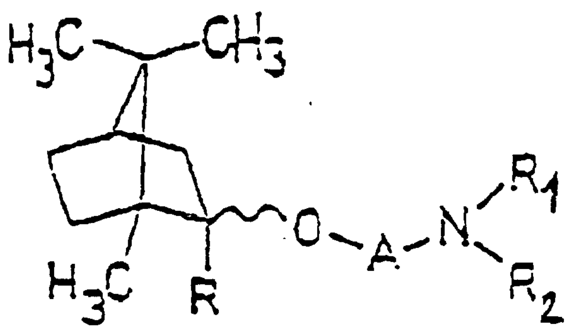

I,

dans laquelle :

| | |
|---|---|
| R | représente un groupe phényle ou benzyle, |
| $R_1$ et $R_2$ | qui peuvent être identiques ou différents représentent des groupes alkyles à chaîne droite ou ramifiée ayant de 1 à 4 atomes de carbone, ou bien l'un des $R_1$ et $R_2$ est un atome d'hydrogène et l'autre est un groupe alkyle à chaîne droite ou ramifiée ayant 1 à 4 atomes de carbone, |
| A | représente un groupe alkylène à chaîne droite ou ramifiée ayant de 2 à 4 atomes de carbone, et |
| - | représente une liaison de valence, |

et d'un ou de N-oxydes, ainsi que d'un ou d'isomères optiques de ceux-ci et de mélanges des isomères optiques et d'un ou de sels d'addition d'acide et d'un ou de dérivés ammonium quaternaire des dérivés de bicycloheptane de formule (I), ainsi que d'un ou d'isomères optiques de ceux-ci et de mélanges des isomères optiques, pour préparer des médicaments pour la prévention ou le traitement d'affections et de troubles qui sont associés à l'influence sur le système de la cholécystokinine périphérique (CCK), choisis parmi les contractions spastiques de la vésicule biliaire et la cholélithiase ainsi que la pancréatite aiguë.

2. Utilisation suivant la revendication 1, **caractérisée en ce qu'**il est utilisé en tant que dérivé ou dérivés de bicycloheptane un ou des dérivés dans lesquels le ou les groupes alkyles qui sont représentés par $R_1$ et/ou $R_2$ ont 1 ou 2 atomes de carbone.

3. Utilisation suivant les revendications 1 ou 2, **caractérisée en ce qu'**il est utilisé en tant que dérivé ou dérivés de bicycloheptane un ou des dérivés dans lesquels le groupe alkylène représenté par A comprend 2 ou 3 atomes de carbone.

4. Utilisation suivant les revendications 1 à 3, **caractérisée en ce qu'**il est utilisé en tant que dérivé ou dérivés de bicycloheptane, le (1R,2S,4R)-(-)-2-[phényl]-2-[2'-(diméthylamino)-éthoxy]-1,7,7-tri-[méthyl]-bicyclo-[2.2.1]-heptane et/ou un ou des sels d'addition d'acide, en particulier le (E)-2-butènedioate de (1R,2S,4R)-(-)-2-[phényl]-2-[2'-(diméthylamino)-éthoxy]-1,7,7-tri-[méthyl]-bicyclo-[2.2.1]-heptane.

**5.** Utilisation suivant les revendications 1 à 4, **caractérisée en ce qu'**il est utilisé en tant que dérivé ou dérivés de bicycloheptane, le (1R,2S,4R)-(-)-2-[benzyl]-2-[3'-(diméthylamino)-propoxy]-1,7,7-tri-[méthyl]-bicyclo-[2.2.1]-heptane, le (1R,2S,4R)-(-)-2-[benzyl]-2-(2'-(méthyl)-3'-(diméthylamino)-propoxy]-1,7,7-tri-[méthyl]-bicyclo-[2.2.1]-heptane, le (1RS,2RS,4RS)-2-[phényl]-2-[2'-(diméthylamino)-éthoxy]-1,7,7-tri-[méthyl]-bicyclo-[2.2.1]-heptane et/ou le (1S,2R,4S)-(+)-2-[phényl]-2-[2'-(diméthylamino)-éthoxy]-1,7,7-tri-[méthyl]-bicyclo-[2.2.1]-heptane et/ou un ou des sels d'addition d'acide de ceux-ci, en particulier le ou les (E)-2-butènedioates.

**6.** Utilisation suivant les revendications 1 à 5, **caractérisée en ce qu'**elle sert à la préparation en tant que médicaments de compositions pharmaceutiques, en particulier pour l'administration par voie orale, rectale ou parentérale.

**7.** Utilisation suivant les revendications 1 à 6, **caractérisée en ce qu'**elle sert à la préparation en tant que médicaments de compositions pharmaceutiques sous la forme de comprimés, de comprimés à revêtement entérique, de gélules, de dragées, de solutions, de suspensions, de suppositoires ou de solutions injectables.

**8.** Utilisation suivant les revendications 1 à 7, **caractérisée en ce qu'**elle sort à la préparation en tant que médicaments de compositions pharmaceutiques qui contiennent de 10 à 100 mg d'un ou de dérivés de bicycloheptane.